# FASCICULE DE BREVET EUROPEEN

(11) **EP 0 916 652 B1**
(45) Date de publication et mention de la délivrance du brevet: **31.10.2001**
(21) Numéro de dépôt: 98402503.1
(22) Date de dépôt: 08.10.1998
(51) Int. Cl.: C07C 271/64, C07C 275/50, A61K 7/06, C07D 295/20

(54) **Nouveaux composés dérivés de n-aryl 2-hydroxy alkylamides**
Neue N-Aryl-2-hydroxy-alkylamide
N-Aryl 2-hydroxy alkylamides

(30) Priorité: 13.11.1997 FR 9714241
(43) Date de publication de la demande: 19.05.1999
(73) Titulaire: L'OREAL, 75008 Paris (FR)
(72) Inventeur: Galey, Jean-Baptiste, 93600 Aulnay-sous-Bois (FR); Destrée, Odile, 77270 Villeparisis (FR); Breton, Lionel, 78000 Versailles (FR)
(74) Mandataire: Tezier Herman, Béatrice

(56) Documents cités:
- EP-A- 0 524 781
- WO-A-97/32562

## Description

La présente invention concerne de nouveaux composés dérivés de N-aryl 2-hydroxy alkylamides.
Elle concerne également leur procédé de synthèse, les compositions les contenant ainsi que l'utilisation d'au moins un de ces composés pour induire et/ou stimuler la croissance des cheveux et/ou freiner leur chute et/ou à traiter l'hyperséborrhée et/ou l'acné.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos en phase télogène et seront donc remplacés en quelques mois.

Cependant différentes causes peuvent entraîner une perte importante, temporaire ou définitive, des cheveux. L'alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

Le terme alopécie recouvre toute une famille d'atteintes du follicule pileux ayant pour conséquence finale la perte définitive partielle ou générale des cheveux. Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

On recherche depuis de nombreuses années, dans l'industrie cosmétique ou pharmaceutique, des substances permettant de supprimer ou de réduire l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-pipéridino pyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.
On peut encore citer les composés appartenant à la famille des N-aryl-2-hydroxyalkylamides qui sont décrits dans la demande de brevet WO97/32562.

Il reste, d'une manière générale, qu'il serait intéressant et utile de pouvoir disposer de composés actifs autres que ceux déjà connus, potentiellement plus actifs et/ou moins toxiques.

Ce but et d'autres sont atteints par la présente invention qui concerne de nouveaux composés répondant à la formule générale (I) 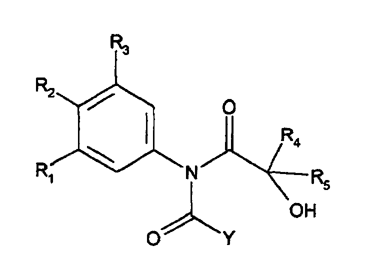 dans laquelle
R₁ représente un atome d'hydrogène, un atome d'halogène, un groupement cyano (-CN), un radical alkyle en C₁ - C₄, un radical perfluoroalkyle ou un radical -OR₆, R₆ représentant un atome d'hydrogène ou un radical alkyle en C₁ - C₄ ;
R₂ représente un groupement nitro (-NO₂), un groupement cyano (-CN), un atome d'halogène, un radical -SO₂R₇, un radical -COR₇ ou un radical -COOR₇, R₇ représentant un atome d'hydrogène, un radical alkyle en C₁-C₄ ou un radical phényle, éventuellement substitué par au moins un atome d'halogène ;
R₃ représente un atome d'hydrogène, un atome d'halogène, un groupement cyano (-CN), un radical alkyle en C₁-C₄, éventuellement substitué par au moins un atome d'halogène ou un radical -OR₆, R₆ représentant un atome d'hydrogène ou un radical alkyle en C₁-C₄ ;
R₄ représente un atome d'hydrogène ou un radical alkyle en C₁-C₄, éventuellement substitué par au moins un atome d'halogène;
R₅ représente un radical alkyle en C₁-C₄ substitué par au moins un atome d'halogène ou un radical phényle éventuellement substitué par au moins un atome d'halogène, un groupement cyano (-CN) ou un groupement nitro (-NO₂);
Y représente un radical OR ou un radical NRR' ;
R, R', identiques ou différents, représentent un atome d'hydrogène, un radical alkyle en C₁-C₈, éventuellement substitué par au moins un radical hydroxy (-OH) ou un radical -COOR₆ ; dans le cas où Y représente NRR', R et R' peuvent former un cycle à 5 ou 6 chaînons.

L'invention concerne également les isomères optiques et/ou géométriques, seuls ou en mélange en toutes proportions, les formes acylées ou encore les sels pharmaceutiquement acceptables de ces composés.

Parmi les atomes d'halogène, on préfère selon l'invention le fluor, le chlore, l'iode ou le brome et plus particulièrement le fluor.

Par radical alkyle en C₁ - C₄ on entend selon l'invention les radicaux acycliques provenant de l'enlèvement d'un atome d'hydrogène dans la molécule d'un hydrocarbure, linéaires ou ramifiés ayant de 1 à 4 atomes de carbone et en particulier les radicaux méthyle, éthyle, propyle, isopropyle, butyle, isobutyle ou tertiobutyle et plus particulièrement le radical méthyle.

Par radical alkyle éventuellement substitué par au moins un atome d'halogène on entend de préférence les radicaux définis ci-dessus pour lesquels au moins un atome d'hydrogène est substitué par un atome d'halogène, y compris les radicaux perhalogénés pour lesquels tous les atomes d'hydrogène sont remplacés par autant d'atomes d'halogènes.

D'une manière préférentielle les radicaux alkyles substitués par au moins un atome d'halogène sont substitués par au moins un atome de chlore ou de fluor. Les radicaux perhalogénés sont préférentiellement des radicaux perfluorés, en particulier des radicaux perfluorométhyles.

Lorsque R₁ est un atome d'halogène, R₁ est préférentiellement un atome de chlore.

Lorsque R₁ est un radical alkyle en C₁ - C₄, R₁ est préférentiellement un radical méthyle.

Lorsque R₁ est un radical perfluoroalkyle, R₁ est préférentiellement un radical trifluorométhyle (-CF₃).

Lorsque R₁ est un radical -OR₆, R₁ est préférentiellement un radical méthoxy (-OCH3).

Lorsque R₂ est un atome d'halogène, R₂ est préférentiellement un atome de chlore.

Lorsque R₂ est un radical -SO₂R₇, R₇ est préférentiellement un radical phényle.

Lorsque R₂ est un radical -COR₇, R₇ est préférentiellement un radical phényle.

Lorsque R₂ est un radical -COOR₇, R₇ est préférentiellement un radical éthyle.

Lorsque R₃ est un atome d'halogène, R₃ est préférentiellement un atome de chlore.

Lorsque R₃ est un radical alkyle en C₁ - C₄, éventuellement substitué par au moins un atome d'halogène, R₃ est préférentiellement un radical trifluorométhyle (-CF₃).

Lorsque R₃ est un radical -OR_{6,} R₆ est préférentiellement un radical méthyle (-CH3).

Lorsque R₄ est un radical alkyle en C₁ - C₄, R₄ est préférentiellement un radical méthyle (-CH3).

Lorsque R₄ est un radical alkyle en C₁ - C₄ substitué par au moins un atome d'halogène, R₄ est préférentiellement un radical trifluorométhyle (-CF3).

Lorsque R₅ est un radical alkyle en C₁ - C₄ substitué par au moins un atome d'halogène, R₅ est préférentiellement un radical trifluorométhyle (-CF₃).

R₄ et R₅ peuvent être identiques mais de préférence R₄ et R₅ ont une signification différente.

Y représente de préférence un radical -NHR.

Lorsque R représente un radical alkyle en C₁-C₈, R est de préférence un radical butyle.

Lorsque R représente un radical -COOR₆, R₆ est de préférence un radical propyle.

On peut citer comme composés de formule (I) :
- l'acide (4-cyano-3-trifluorométhyl-phényl)-(3,3,3-trifluoro-2-hydroxy-2-méthyl propionyl)-carbamique ;
- l'ester éthylique de l'acide (4-cyano-3-trifluorométhyl-phényl)-(3,3,3-trifluoro-2-hydroxy-2-méthyl propionyl)-carbamique ;
- le 1-(3,4-dichloro-phényl)-3-propyl-1-(3,3,3-trifluoro-2-hydroxy-2-méthylpropionyl)-urée ;
- le 1-(3,4-dichloro-phényl)-3-benzyl-1-(3,3,3-trifluoro-2-hydroxy-2-méthylproplonyl)-urée ;
- le 1-(3,4-dichloro-phényl)-3-piperidinyl-1-(3,3,3-trifluoro-2-hydroxy-2-méthylpropionyl)-urée ;
- le 1-(4-sulfonyl-phényl)-3-propyl-1-(3,3,3-trifluoro-2-hydroxy-2-méthylpropionyl)-urée ;
- le 1-(4-benzoyl-phényl)-3-propyl-1-(3,3,3-trifluoro-2-hydroxy-2-méthylpropionyl)-urée ;
- l'ester éthylique de l'acide (3,4-dichloro-phényl)-(3,3,3-trifluoro-2-hydroxy-2-méthyl propionyl)-carbamique ;
- le 1-(3,4-dichloro-phényl)-3-(4-hydroxy-butyl)-1-(3,3,3-trifluoro-2-hydroxy-2-méthyl-propionyl)-urée ;

Parmi ces composés on préfère tout particulièrement :
- l'acide (4-cyano-3-trifluorométhyl-phényl)-(3,3,3-trifluoro-2-hydroxy-2-méthyl propionyl)-carbamique ;
- l'ester éthylique de l'acide (4-cyano-3-trifluorométhyl-phényl)-(3,3,3-trifluoro-2-hydroxy-2-méthyl propionyl)-carbamique ;
- le 1-(3,4-dichloro-phényl)-3-propyl-1-(3,3,3-trifluoro-2-hydroxy-2-méthylpropionyl)-urée ; Un deuxième objet de l'invention est un procédé de préparation des composés de formule (I) tels que définis ci-dessus.

Ce procédé est caractérisé par le fait que l'on prépare, dans une première étape, un dérivé N-aryle 2,4-dioxo-oxazoiidine selon le procédé décrit par Patton, T.L. J. Org. Chem. 1967, 32(2) :383-388, en faisant réagir dans un solvant anhydre un isocyanate avec une cyanhydrine en présence d'une amine. Le mélange obtenu est traité en présence d'acide chlorhydrique, puis le dérivé N-aryle 2,4-dioxooxazolidine formé est alors extrait à l'aide d'un solvant organique, puis séché et purifié par chromatographie sur colonne de silice. Dans une deuxième étape, on ajoute un excès d'alcool ou d'amine à une solution de N-aryle 2,4-dioxooxazolidine obtenu dans la première étape. La solution est chauffée entre 25°C et 70°C pendant 1 à 7 jours puis évaporée à sec. Le résidu est purifié par chromatographie sur colonne de silice ou recristallisé.

Des exemples détaillés de préparation des composés selon l'invention est donné par ailleurs dans les exemples.

Un troisième objet de l'invention concerne des compositions cosmétiques ou pharmaceutiques, particulièrement dermatologiques, qui comprennent au moins un des composés répondant à la formule (I) définis ci-dessus.

Bien entendu les compositions selon l'invention peuvent comprendre les composés de formule (I) seuls ou en mélanges en toutes proportions.

La quantité de composés de formule (I) contenue dans les compositions de l'invention est bien entendu fonction de l'effet recherché et peut donc varier dans une large mesure.

Pour donner un ordre de grandeur, si la composition est une composition cosmétique, elle peut contenir au moins un composé de formule (I) en une quantité représentant de 0.0001% à 5% du poids total de la composition et préférentiellement en une quantité représentant de 0.001% à 2% du poids total de la composition.

Pour donner un ordre de grandeur, si la composition est une composition pharmaceutique, elle peut contenir au moins un composé de formule (I) en une quantité représentant de 0.001% à 10% du poids total de la composition et préférentiellement en une quantité représentant de 0.01% à 5 % du poids total de la composition.

La composition peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins composé de formule (I) à d'autres agents actifs. Parmi ces agents actifs, on peut citer à titre d'exemple :
- les agents améliorant l'activité sur la repousse et/ou sur le freinage de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale ou bactérienne.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétrayénoïque et eicosatriyénoïque ou leurs esters et amides, la vitamine D et ses dérivés, des extraits d'origine végétale ou bactérienne.
Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

On peut également envisager que la composition comprenant au moins un composé tel que défini précédement soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition pharmaceutique selon l'invention peut être administrée par voie parentérale, entérale ou encore par voie topique. De préférence, la composition pharmaceutique est administrée par voie topique.

Un quatrième objet de l'invention concerne l'utilisation à titre de principe actif, dans un milieu physiologiquement acceptable, dans une composition cosmétique ou pour la préparation d'une composition pharmaceutique, d'une quantité efficace d'au moins un composé de formule (I) définis ci-dessus.

Les composés de formule (I) sont en effet d'excellents ouvreurs de canaux potassiques, propriété principale du Minoxidil, seul composé reconnu à ce jour comme efficace dans les traitements de la chute des cheveux.

Ils sont également d'excellents antagonistes réceptoriels des androgènes, les androgènes étant responsables d'une forme particulièrement répandue de l'alopécie, l'alopécie androgéno-dépendante. Mais, on sait également que les androgènes sont impliqués dans l'hyperséborrhée et l'acné.

Ainsi, les composés de formule (I) présentent des activités remarquables qui justifient leur utilisation à titre de médicament, en particulier pour induire et/ou stimuler la croissance des cheveux et/ou freiner leur chute et/ou dans le traitement de l'hyperséborrhée et/ou de l'acné.

A la connaissance de la demanderesse il n'a jamais été proposé dans l'art antérieur l'utilisation de tels composés dérivés de N-aryl 2-hydroxy alkylamides à activité mixte, ouvreurs de canaux potassiques / anti-androgènes, pour lutter contre la chute des cheveux et/ou dans le traitement de l'hyperséborrhée et/ou de l'acné.

Ainsi, l'invention à pour objet l'utilisation à titre de principe actif, dans un milieu physiologiquement acceptable, dans une composition cosmétique ou pour la préparation d'une composition pharmaceutique, d'une quantité efficace d'au moins un composé de formule (I), ce composé ou les compositions étant destinés à induire et/ou stimuler la croissance des cheveux et/ou freiner leur chute et/ou à traiter l'hyperséborrhée et/ou l'acné.

Bien entendu les composés peuvent être utilisés seuls ou en mélange.

La composition cosmétique selon l'invention est à appliquer sur les zones du corps nécessitant un traitement, comme par exemple les zones alopéciques du cuir chevelu ou les cheveux d'un individu. La composition cosmétique peut alors éventuellement être laissée en contact plusieurs heures puis est éventuellement rincée. On peut, par exemple, appliquer la composition contenant une quantité efficace d'au moins un composé tel que défini précédement, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu et/ou de la peau, caractérisé par le fait qu'il consiste à appliquer sur les cheveux et/ou le cuir chevelu et/ou la peau, une composition cosmétique comprenant une quantité efficace d'au moins composé de formule (I), à laisser celle-ci en contact avec les cheveux et/ou le cuir chevelu et/ou la peau, et éventuellement à rincer.

Le procédé de traitement présente les caractéristiques d'un procédé cosmétique dans la mesure où il permet d'améliorer l'esthétique des cheveux et/ou de la peau en leur donnant une plus grande vigueur et un aspect amélioré.

On va maintenant donner à titre d'illustration des exemples qui ne sauraient limiter en aucune façon la portée de l'invention.

### Exemple 1 : Synthèse de l'ester éthylique de l'acide (4-cyano-3-trifluorométhylphényl)-(3,3,3-trifluoro-2-hydroxy-2-méthyl propionyl)-carbamique de formule :

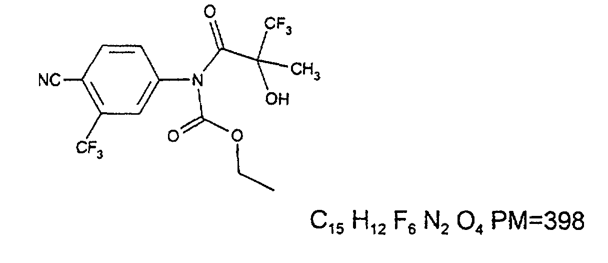

### a) Synthèse du 4-(5-méthyl-2,4-dioxo-5-trifluorométhyl)-oxazolidin-3-yl)-2-trifluorométhylbenzonitrile

On dissout 1.09 g de 4-isocyanato 2-trifluorométhyl benzonitrile dans 20 ml de THF anhydre. On additionne 150 µl de Triéthylamine puis 860 mg de trifluoroacétone cyanohydrine en solution dans 10 ml de THF anhydre. Le milieu réactionnel est agité 2h à température ambiante.

On additionne 5 ml de méthanol puis après 30 mn, 10 ml d'acide chlorhydrique 1N. Après 2 h à température ambiante, on ajoute 20 ml d'eau. Le milieu réactionnel est extrait par 4 x 25 ml de dichlorométhane. La phase organique est lavée par 25 ml d'eau, séchée sur sulfate de sodium, évaporée à sec. Le résidu obtenu est purifié par chromatographie sur colonne de silice, l'éluant étant du dichlorométhane. On obtient 540 mg d'un précipité blanc.

### b) : Synthèse de l'ester éthylique de l'acide (4-cyano-3-trifluorométhylphényl)-(3,3,3-trifluoro-2-hydroxy-2-méthyl propionyl)-carbamique :

4,3 g de 4-(5-méthyl-2,4-dioxo-5-trifluorométhyl-oxazolidin-3-yl)-2-trifluorométhylbenzonitrile sont solubilisés dans 50 ml d'éthanol. La solution est portée au reflux 7 jours puis évaporée à sec. Le résidu est purifié par chromatographie sur colonne de silice (éluant : dichlorométhane) puis recristallisé dans un mélange heptane 2 / toluène 1.

On obtient 1,4 g d'ester éthylique de l'acide (4-cyano-3-trifluorométhyl-phényl)-(3,3,3-trifluoro-2-hydroxy-2-méthyl propionyl)-carbamique, pour un rendement de 29 %.

### Analyses :

RMN ¹H (dans CD₃CN) : d (ppm) = 1.23 (t, 3H) ; 1.87 (d, 3H) ; 4.27 (q, 2H) ; 7.75 (dd, 1H) ; 7.88 (d, 1H) ; 7.94 (d, 1H) ; 8.93 (s, 1H)
RMN ¹³C (dans CD₃CN) : d (ppm) = 14.14 ; 17.51 ; 64.17 ; 81.06 ; 104.26; 116.66 ; 117.34 ; 123.60 ; 123.99 ; 134.12 ; 137.38 ; 143.66 ; 151.49 ; 165.97

| Analyse élémentaire : | | | | |
|---|---|---|---|---|
| | C | H | N | F |
| %Calculé | 45.23 | 3.01 | 7.03 | 28.64 |
| %Trouvé | 45.21 | 3.09 | 6.86 | 28.48 |

### Exemple 2 : Synthèse de l'acide (4-cyano-3-trifluorométhyl-phényl)-(3,3,3-trifluoro-2-hydroxy-2-méthyl propionyl)-carbamique de formule :

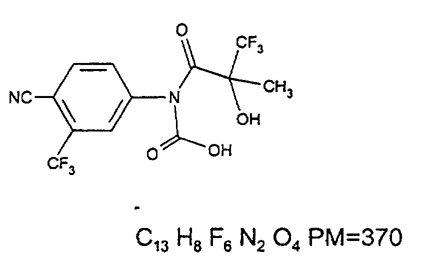

100 mg de 4-(5-méthyl-2,4-dioxo-5-trifluorométhyl-oxazolidin-3-yl)-2-trifluorométhyl-benzonitrile tel que préparé dans l'exemple 1, sont solubilisés dans 20 ml dans un mélange éthanol/eau 7 :3 et agités 5 jours à température ambiante. Le milieu réactionnel est évaporé à sec puis purifié par chromatographie sur colonne de silice RP18 (éluant : Eau 6 / Acétonitrile 4). On obtient 18 mg d'acide (4-cyano-3-trifluorométhyl-phényl)-(3,3,3-trifluoro-2-hydroxy-2-méthyl-propionyl)-carbamique, pour un rendement de 17%.

### ANALYSES :

RMN ¹H (dans CD₃CN) : d (ppm) = 1.86 (s, 3H) ; 7.75 (dd, 1H) ; 7.86 (d, 1H) ; 7.95 (d, 1H) ; 8.75 (s, 1H)
RMN ¹H (dans DMSO d6) : d (ppm) = 1.81 (s, 3H) ; 7.84 (dd, 1H) ; 8.08 (d, 1H) ; 8.10 (d, 1H) ; 10.88 (s, 1H) ; 14.36 (s, OH)
RMN ¹³C (dans CD₃CN) : d (ppm) = 16.36 ; 104.15 ; 116.68 ; 117.28 ; 122.36 ; 134.12 ; 137.54 ; 143.72 ; 151.52 ; 166.97
RMN ¹⁹F (dans CD₃CN) : d (ppm) = -61.00 (CF₃) ; -77.12 (F11)

### Exemple n°3 : Synthèse du 1-(3,4-dichloro-phényl)-3-propyl-1-(3,3,3-trifluoro-2-hydroxy-2-méthyl-propionyl)-urée de formule :

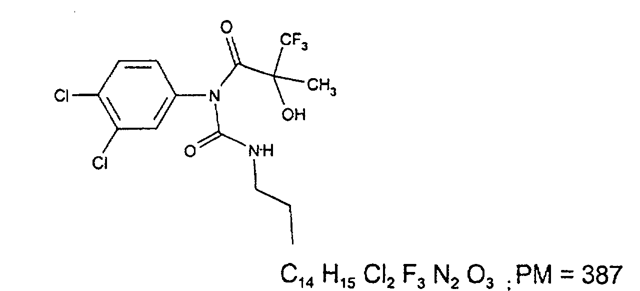

### a) Synthèse du 3-(3,4-dichlorophényl)-5-méthyl-5-trifluorométhyloxazolidine-2,4-dione.

3 g de N-(3,4-dichloro-phényl)-3,3,3-trifluoro-2-hydroxy-2-méthyl-propionamide sont solubilisés dans 50 ml de THF préalablement distillé sur sodium/benzophénone. On ajoute 1 g de l'hydrure de sodium (NaH) à 60% dans l'huile. Le milieu réactionnel est agité 30 mn puis refroidi à 0-5°. On ajoute 2,3 g de chloroformiate d'éthyle en solution dans 10 ml de THF, sans excéder 5°C. Après une nuit à température ambiante, le milieu réactionnel est coulé sur 100 ml d'eau glacée puis extrait par 4x50 ml de dichlorométhane. La phase organique est lavée par du chlorure de sodium à 20%, séchée sur sulfate de sodium, évaporée à sec. Le résidu est purifié par chromatographie sur silice (Eluant Heptane 1/ Dichloromethane 1). On obtient 1,25g de 3-(3,4-Dichlorophényl)-5-méthyl-5-Trifluorométhyl-oxazolidine-2,4-dione pour un rendement de 38%.

### ANALYSES :

RMN ¹H (dans DMSO d6) : d (ppm) = 1.96 (s, 3H) ; 7.57 (dd, 1H) ; 7.88 (d, 1H) ; 7.90 (d, 1H)
RMN ¹³C (dans DMSO d6) : d (ppm) = 15.05 ; 81.84 ; 121.49 ; 126.78 ; 128.35 ; 129.75 ; 131.35 ; 131.62 132.46 ; 150.98 ; 165.95

| Analyse élémentaire : | | | | | |
|---|---|---|---|---|---|
| | C | H | Cl | F | N |
| %Calculé | 40.24 | 1.83 | 21.65 | 17.38 | 4.27 |
| %Trouvé | 40.29 | 1.96 | 21.70 | 17.48 | 4.35 |

### b) Synthèse du 1-(3,4-dichloro-phényl)-3-propyl-1-(3,3,3-trifluoro-2-hydroxy-2-méthyl-propionyl)-urée :

200 mg de 3-(3,4-dichlorophényl)-5-méthyl-5-trifluorométhyl-oxazolidine-2,4-dione sont solubilisés dans 10 ml de dichlorométhane. On ajoute 55 µl de propylamine. Le milieu réactionnel est agité pendant 24 heures à température ambiante. Après évaporation, le résidu est purifié par chromatographie sur colonne de silice (éluant dichlorométhane).
On obtient 155 mg de 1-(3,4-dichloro-phényl)-3-propyl-1-(3,3,3-trifluoro-2-hydroxy-2-méthyl-propionyl)-urée, pour un rendement de 63%.

### ANALYSES

RMN ¹H (dans DMSO d6) : d (ppm) = 0.84 (t, 3H) ; 1.43 (m, 2H) ; 1.81 (s, 3H) ; 2.94 (m, 2H) ; 7.59 (d, 1H) ; 7.65 (dd, 1H) ; 7.69 (t, 1H) ; 7.95 (d, 1H) ; 10.17 (s, OH)

### Exemple 4 : Mesure de l'affinité (IC 50) de l'ester éthylique de l'acide (4-cyano-3-trifluorométhyl-phényl)-(3,3,3-trifluoro-2-hydroxy-2-méthyl propionyl)-carbamique (ex. 1) et de l'acide (4-cyano-3-trifluorométhyl-phényl)-(3,3,3-trifluoro-2-hydroxy-2-méthyl propionyl)-carbamique (ex. 2) pour le récepteur aux androgènes.

Ces mesures d'affinité réceptorielle pour le récepteur aux androgènes sont effectuées selon la méthode de Schilling et Liao, décrite dans "The Prostate", 1984, 5, p. 581-588.

Au cours de l'expérimentation, la molécule de référence (mibolerone) est parallèlement testée à 8 concentrations afin de valider l'expérience.

| n° ex | inhibition de la fixation de la testostérone : IC50 (µM) |
|---|---|
| 1 | 2 |
| 2 | 4 |

La référence interne (mibolerone) inhibe de 50 % la fixation de la testostérone à son récepteur à une concentration de 4,3 nM.

### Exemple 5 : Mesure de l'activité de type "ouvreur de canaux potassiques" de l'ester éthylique de l'acide (4-cyano-3-trifluorométhyl-phényl)-(3,3,3-trifluoro-2-hydroxy-2-méthyl propionyl)-carbamique (ex. 1) et de l'acide (4-cyano-3-trifluorométhyl-phényl)-(3,3,3-trifluoro-2-hydroxy-2-méthyl propionyl)-carbamique (ex. 2), par la mesure in vitro du pouvoir relaxant des molécules sur des anneaux d'aorte thoracique (IC50).

Les expériences sont réalisées selon les méthodes de Newgreen et al (Br. J. Pharmacol., 100, 1990, p. 605-613), Bray et al (Arch. Pharmacol., 344, 1991, p. 351-359) et Wickerden et al (Br. J. Pharmacol., 103, 1991, 1148-1152).

Après leur installation dans les cuves à organes isolés, les tissus (muscles lisses aortiques) sont soumis à une tension initiale de 2 g.

Après une période d'équilibration, les tissus sont exposés à une solution de chlorure de potassium (KCI, à 20 mM) pour obtenir une réponse contractile soutenue.
Après stabilisation de cette réponse contractile, l'activité relaxante (de type ouvreur de canaux potassiques) des molécules à tester est évaluée en fonction de la concentration appliquée.

Au cours de l'expérimentation, deux molécules références sont utilisées : la cromakalim et le Minoxidil. Elle présentent des IC50 de l'ordre de 1 µM.

| n° ex | inhibition contraction aorte isolée induite par K⁺ : IC50 (µM) |
|---|---|
| 1 | 2 |
| 2 | 3 |

Les résultats montrent que les composés des exemples 1 et 2 présentent une activité mixte.

### Exemple 6 :

Exemples de compositions contenant un dérivé de N-aryl 2-hydroxy alkylamide. Ces compositions sont obtenues par les techniques habituelles couramment utilisées en cosmétique ou en pharmacie.

| Lotion antichute: | |
|---|---|
| Composé de l'exemple 3 | 1,000 g |
| Propylène glycol | 30,000 g |
| Alcool éthylique | 40,500 g |
| Eau | qsp 100,000 g |

On applique cette lotion sur le cuir chevelu, une à deux fois par jour, à raison d'1 ml par application.

| Lotion antichute épaissie: | |
|---|---|
| Composé de l'exemple 2 | 0,500 g |
| Kawaïne | 2,000 g |
| Hydroxypropylcellulose* | 3,500 g |
| Alcool éthylique | qsp 100,000 g |

| | |
|---|---|
| * : Klucel G® de la société Hercules | |

On applique cette lotion épaissie sur le cuir chevelu, une à deux fois par jour, à raison d'1 ml par application.

| lotion antichute: | |
|---|---|
| Composé de l'exemple 1 | 0,200 g |
| Monométhyléther de propylèneglycol** | 20,000 g |
| Hydroxypropylcellulose* | 3,000 g |
| Alcool éthylique | 40,000 g |
| Minoxidil | 2,000 g |
| Eau | qsp 100,000 g |

| | |
|---|---|
| * : Klucel G® de la société Hercules | |
| **: Dowanol PM® de Dow Chemical | |

On applique cette lotion épaissie sur le cuir chevelu, une à deux fois par jour, à raison d'1 ml par application.

| Lotion antichute: | |
|---|---|
| Composé de l'exemple 3 | 0,100 g |
| Propylène glycol | 10,000 g |
| Alcool isopropylique | qsp 100,000 g |

On applique 1 ml de cette lotion sur le cuir chevelu, à la fréquence de une à deux fois par jour.

Avec chacune des compositions décrites dans les exemples ci-dessus, on a constaté, après plusieurs mois de traitement et selon les sujets traités, un ralentissement de la chute des cheveux et/ou un effet repousse.

## Revendications

1. Composés répondant à la formule générale (I): 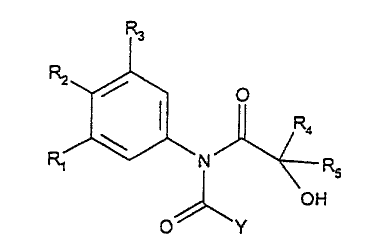 dans laquelle
R₁ représente un atome d'hydrogène, un atome d'halogène, un groupement cyano (-CN), un radical alkyle en C₁ - C₄, un radical perfluoroalkyle ou un radical -OR₆, R₆ représentant un atome d'hydrogène ou un radical alkyle en C₁ - C₄ ;
R₂ représente un groupement nitro (-NO₂), un groupement cyano (-CN), un atome d'halogène, un radical -SO₂R₇, un radical -COR₇ ou un radical -COOR₇, R₇ représentant un atome d'hydrogène, un radical alkyle en C₁-C₄ ou un radical phényle, éventuellement substitué par au moins un atome d'halogène ;
R₃ représente un atome d'hydrogène, un atome d'halogène, un groupement cyano (-CN), un radical alkyle en C₁-C₄, éventuellement substitué par au moins un atome d'halogène ou un radical -OR₆, R₆ représentant un atome d'hydrogène ou un radical alkyle en C₁-C₄ ;
R₄ représente un atome d'hydrogène ou un radical alkyle en C₁-C₄, éventuellement substitué par au moins un atome d'halogène ;
R₅ représente un radical alkyle en C₁-C₄ substitué par au moins un atome d'halogène ou un radical phényle éventuellement substitué par au moins un atome d'halogène, un groupement cyano (-CN) ou un groupement nitro (-NO₂) ;
Y représente un radical OR ou un radical NRR' ;
R, R', identiques ou différents, représentent un atome d'hydrogène, un radical alkyle en C₁-C₈, éventuellement substitué par au moins un radical hydroxy (-OH) ou un radical -COOR₆ ; dans le cas où Y représente NRR', R et R' peuvent former un cycle à 5 ou 6 chaînons.
ou ses isomères optiques et/ou géométriques, seuls ou en mélange en toutes proportions, ses formes acylées ou encore ses sels pharmaceutiquement acceptables.

2. Composés selon la revendication précédente, **caractérisés par le fait que** R₁ est un atome de chlore.

3. Composés selon la revendication 1, **caractérisés par le fait que** R, est préférentiellement un radical méthyle.

4. Composés selon la revendication 1, **caractérisés par le fait que** R₁ est un radical trifluorométhyle (-CF₃).

5. Composés selon la revendication 1, **caractérisés par le fait que** R₁ est un radical méthoxy (-OCH3).

6. Composés selon l'une quelconque des revendications précédentes, **caractérisés par le fait que** R₂ est un atome de chlore.

7. Composés selon l'une quelconque des revendications 1 à 5, **caractérisés par le fait que** R₂ est un radical -SO₂R₇ dans lequel R₇ est un radical phényle.

8. Composés selon l'une quelconque des revendications 1 à 5, **caractérisés par le fait que** R₂ est un radical -COR₇ dans lequel R₇ est un radical phényle.

9. Composés selon l'une quelconque des revendications 1 à 5, **caractérisés par le fait que** R₂ est un radical -COOR₇ dans lequel R₇ est un radical éthyle.

10. Composés selon l'une quelconque des revendications précédentes, **caractérisés par le fait que** R₃ est un atome de chlore.

11. Composés selon l'une quelconque des revendications 1 à 9, **caractérisés par le fait que** R₃ est un radical trifluorométhyle (-CF₃).

12. Composés selon l'une quelconque des revendications 1 à 9, **caractérisés par le fait que** R₃ est un radical -OR₆ dans lequel R₆ est un radical méthyle (-CH3).

13. Composés selon l'une quelconque des revendications précédentes, **caractérisés par le fait que** R₄ est un radical méthyle (-CH3).

14. Composés selon l'une quelconque des revendications 1 à 12, **caractérisés par le fait que** R₄ est un radical un radical trifluorométhyle (-CF3).

15. Composés selon l'une quelconque des revendications précédentes, **caractérisés par le fait que** R₅ est un radical trifluorométhyle (-CF₃).

16. Composés selon l'une quelconque des revendications précédentes, **caractérisés par le fait que** Y est un radical NHR.

17. Composés selon l'une quelconque des revendications précédentes, **caractérisés par le fait que** R est un radical butyle.

18. Composés selon l'une quelconque des revendications 1 à 16, **caractérisés par le fait que** R est radical un radical -COOR₆ dans lequel R₆ est un radical propyle.

19. Composés selon l'une quelconque des revendications 1 à 18 choisis parmi :
- l'acide (4-cyano-3-trifluorométhyl-phényl)-(3,3,3-trifluoro-2-hydroxy-2-méthyl propionyl)-carbamique ;
- l'ester éthylique de l'acide (4-cyano-3-trifluorométhyl-phényl)-(3,3,3-trifluoro-2-hydroxy-2-méthyl propionyl)-carbamique ;
- le 1-(3,4-dichloro-phényl)-3-propyl-1-(3,3,3-trifluoro-2-hydroxy-2-méthylpropionyl)-urée ;
- le 1-(3,4-dichloro-phényl)-3-benzyl-1-(3,3,3-trifluoro-2-hydroxy-2-méthylpropionyl)-urée ;
- le 1-(3,4-dichloro-phényl)-3-piperidinyl-1-(3,3,3-trifluoro-2-hydroxy-2-méthylpropionyl)-urée ;
- le 1-(4-sulfonyl-phényl)-3-propyl-1-(3,3,3-trifluoro-2-hydroxy-2-méthylpropionyl)-urée ;
- le 1-(4-benzoyl-phényl)-3-propyl-1-(3,3,3-trifluoro-2-hydroxy-2-méthylpropionyl)-urée ;
- l'ester éthylique de l'acide (3,4-dichloro-phényl)-(3,3,3-trifluoro-2-hydroxy-2-méthyl propionyl)-carbamique ;
- le 1-(3,4-dichloro-phényl)-3-(4-hydroxy-butyl)-1-(3,3,3-trifluoro-2-hydroxy-2-méthyl-propionyl)-urée ;

20. Composés selon l'une quelconque des revendications 1 à 19 choisis parmi :
- l'acide (4-cyano-3-trifluorométhyl-phényl)-(3,3,3-trifluoro-2-hydroxy-2-méthyl propionyl)-carbamique ;
- l'ester éthylique de l'acide (4-cyano-3-trifluorométhyl-phényl)-(3,3,3-trifluoro-2-hydroxy-2-méthyl propionyl)-carbamique ;
- le 1-(3,4-dichloro-phényl)-3-propyl-1-(3,3,3-trifluoro-2-hydroxy-2-méthylpropionyl)-urée ;

21. Procédé de préparation des composés de formule (I) tels que définis selon l'une quelconque des revendications 1 à 20, **caractérisé par le fait qu'**il comprend une première étape dans laquelle on fait réagir dans un solvant anhydre un isocyanate avec une cyanhydrine en présence d'une amine, le mélange obtenu étant ensuite traité en présence d'acide chlorhydrique, le dérivé N-aryle 2,4-dioxooxazolidine ainsi formé étant alors extrait à l'aide d'un solvant organique puis séché et purifié par chromatographie sur colonne de silice ;
une deuxième étape dans laquelle on ajoute un excès d'alcool ou d'amine à une solution de N-aryle 2,4-dioxo-oxazolidine obtenu dans la première étape la solution étant alors chauffée entre 25°C et 70°C pendant 1 à 7 jours puis évaporée à sec, le résidu obtenu étant purifié par chromatographie sur colonne de silice ou recristallisé.

22. Composition comprenant au moins un composé de formule (I) tel que défini selon l'une quelconque des revendications 1 à 20.

23. Composition selon la revendication 22, **caractérisée par le fait qu'**elle est destinée à un usage cosmétique ou pharmaceutique..

24. Composition cosmétique selon l'une quelconque des revendications 22 ou 23, **caractérisée par le fait qu'**elle comprend au moins un composé de formule (I) en une quantité représentant de 0.0001% à 5% du poids total de la composition et préférentiellement en une quantité représentant de 0.001% à 2% du poids total de la composition.

25. Composition pharmaceutique selon l'une quelconque des revendications 22 ou 23, **caractérisée par le fait qu'**elle comprend au moins un composé de formule (I) en une quantité représentant de 0.001% à 10% du poids total de la composition et préférentiellement en une quantité représentant de 0.01% à 5 % du poids total de la composition.

26. Composition selon l'une quelconque des revendications 22 à 25, **caractérisée par le fait qu'**elle comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

27. Composition selon l'une quelconque des revendications 22 à 26, **caractérisée par le fait qu'**elle est destinée à induire et/ou stimuler la croissance des cheveux et/ou freiner leur chute et/ou à traiter l'hyperséborrhée et/ou l'acné.

28. A titre de médicaments les composés de formules générale (I) 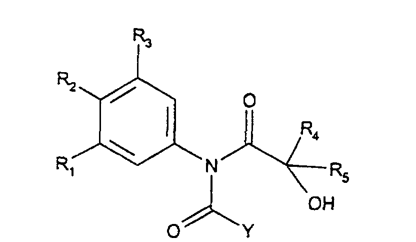 tels que définis dans les revendications 1 à 20.

29. Utilisation dans une composition cosmétique ou pour la préparation d'une composition pharmaceutique, d'une quantité efficace d'au moins un composé répondant à la formule générale (I) tel que défini selon l'une quelconque des revendications 1 à 20, ce composé ou cette composition étant destiné à induire et/ou stimuler la croissance des cheveux et/ou freiner leur chute et/ou traiter l'hyperséborrhée et/ou l'acné.

30. Procédé de traitement cosmétique des cheveux et/ou du cuir chevelu, **caractérisé par le fait qu'**il consiste à appliquer sur les cheveux et/ou le cuir chevelu et/ou sur la peau, une composition cosmétique telle que définie dans l'une quelconque des revendications 22 à 24 ou 26 ou 27, à laisser celle-ci en contact avec les cheveux et/ou le cuir chevelu et/ou la peau, et éventuellement à rincer.

## Patentansprüche

1. Verbindungen der allgemeinen Formel (I): 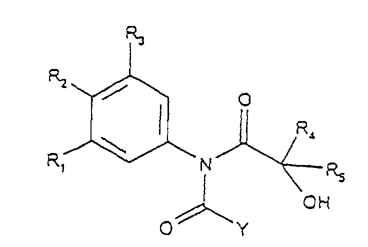 worin bedeuten:
R₁ Wasserstoff, Halogen, Cyano (-CN), C₁₋₄-Alkyl, Perfluoralkyl oder -OR₆, wobei R₆ Wasserstoff oder eine C₁₋₄-Alkylgruppe bedeutet;
R₂ Nitro (-NO₂), Cyano (-CN), Halogen, -SO₂R₇, -COR₇ oder -COOR₇, wobei R₇ Wasserstoff, eine C₁₋₄-Alkylgruppe oder eine Phenylgruppe bedeutet, die gegebenenfalls mit mindestens einem Halogenatom substituiert ist;
R₃ Wasserstoff, Halogen, Cyano (-CN), eine C₁₋₄-Alkylgruppe, die gegebenenfalls mit mindestens einem Halogenatom substituiert ist, oder -OR₆, wobei R₆ Wasserstoff oder eine C₁₋₄-Alkylgruppe bedeutet;
R₄ Wasserstoff oder eine C₁₋₄-Alkylgruppe, die gegebenenfalls mit mindestens einem Halogenatom substituiert ist;
R₅ eine C₁₋₄-Alkylgruppe, die mit mindestens einem Halogenatom substituiert ist, eine Phenylgruppe, die gegebenenfalls mit mindestens einem Halogenatom substituiert ist, eine Cyanogruppe (-CN) oder eine Nitrogruppe (-NO₂);
Y eine Gruppe OR oder eine Gruppe NRR'; und
R und R', die identisch oder voneinander verschieden sind, Wasserstoff, eine C₁₋₈-Alkylgruppe, die gegebenenfalls mit mindestens einer Hydroxygruppe (-OH) substituiert ist, oder -COOR₆; wenn Y eine Gruppe NRR' bedeutet, können R und R' einen 5- oder 6-gliedrigen Ring bilden;
oder ihre optischen und/oder geometrischen Isomere einzeln oder im Gemisch in beliebigen Mengenanteilen, die acylierten Formen oder die pharmazeutisch akzeptablen Salze dieser Verbindungen.

2. Verbindungen nach dem vorhergehenden Anspruch, **dadurch gekennzeichnet, daß** R₁ Chlor bedeutet.

3. Verbindungen nach Anspruch 1, **dadurch gekennzeichnet, daß** R₁ vorzugsweise Methyl bedeutet.

4. Verbindungen nach Anspruch 1, **dadurch gekennzeichnet, daß** R₁ Trifluormethyl (-CF₃) ist

5. Verbindungen nach Anspruch 1, **dadurch gekennzeichnet, daß** R₁ Methoxy (-OCH₃) ist.

6. Verbindungen nach einem der vorhergehenden Ansprüche, **dadurch gekennzeichnet, daß** R₂ Chlor bedeutet.

7. Verbindungen nach einem der Ansprüche 1 bis 5, **dadurch gekennzeichnet, daß** R₂ eine Gruppe -SO₂R₇ ist, worin R₇ Phenyl bedeutet.

8. Verbindungen nach einem der Ansprüche 1 bis 5, **dadurch gekennzeichnet, daß** R₂ eine Gruppe -COR₇ ist, worin R₇ Phenyl bedeutet.

9. Verbindungen nach einem der Ansprüche 1 bis 5, **dadurch gekennzeichnet, daß** R₂ eine Gruppe -COOR₇ ist, worin R₇ Ethyl bedeutet.

10. Verbindungen nach einem der vorhergehenden Ansprüche, **dadurch gekennzeichnet, daß** R₃ Chlor bedeutet.

11. Verbindungen nach einem der Ansprüche 1 bis 9, **dadurch gekennzeichnet, daß** R₃ Trifluormethyl (-CF₃) bedeutet.

12. Verbindungen nach einem der Ansprüche 1 bis 9, **dadurch gekennzeichnet, daß** R₃ eine Gruppe -OR₆ bedeutet, worin R₆ Methyl (-CH₃) ist.

13. Verbindungen nach einem der vorhergehenden Ansprüche, **dadurch gekennzeichnet, daß** R₄ Methyl (-CH₃) bedeutet.

14. Verbindungen nach einem der Ansprüche 1 bis 12, **dadurch gekennzeichnet, daß** R₄ eine Trifluormethylgruppe (-CF₃) ist.

15. Verbindungen nach einem der vorhergehenden Ansprüche, **dadurch gekennzeichnet, daß** R₅ eine Trifluormethylgruppe (-CF₃) ist.

16. Verbindungen nach einem der vorhergehenden Ansprüche, **dadurch gekennzeichnet, daß** Y eine Gruppe -NHR bedeutet.

17. Verbindungen nach einem der vorhergehenden Ansprüche, **dadurch gekennzeichnet, daß** R eine Butylgruppe ist.

18. Verbindungen nach einem der Ansprüche 1 bis 16, **dadurch gekennzeichnet, daß** R eine Gruppe -COOR₆ ist, wobei R₆ Propyl bedeutet.

19. Verbindungen nach einem der Ansprüche 1 bis 18, **dadurch gekennzeichnet, daß** sie ausgewählt sind unter:
- (4-Cyano-3-trifluormethyl-phenyl)-(3,3,3-trifluor-2-hydroxy-2-methyl-propionyl)-carbamidsäure;
- (4-Cyano-3-trifluormethyl-phenyl)-(3,3,3-trifluor-2-hydroxy-2-methyl-propionyl)-carbamidsäureethylester;
- 1-(3,4-Dichlor-phenyl)-3-propyl-1-(3,3,3-trifluor-2-hydroxy-2-methyl-propionyl)-harnstoff;
- 1-(3,4-Dichlor-phenyl)-3-benzyl-1-(3,3,3-trifluor-2-hydroxy-2-methyl-propionyl)-harnstoff;
- 1-(3,4-Dichlor-phenyl)-3-piperidinyl-1-(3,3,3-trifluor-2-hydroxy-2-methyl-propionyl)-harnstoff;
- 1-(4-Sulfonyl-phenyl)-3-propyl-1-(3,3,3-trifluor-2-hydroxy-2-methyl-propionyl)-harnstoff;
- 1-(4-Benzoyl-phenyl)-3-propyl-1-(3,3,3-trifluor-2-hydroxy-2-methyl-propionyl)-harnstoff;
- (3,4-Dichlor-phenyl)-(3,3,3-trifluor-2-hydroxy-2-methylpropionyl)-carbamidsäureethylester; und
- 1-(3,4-Dichlor-phenyl)-3-(4-hydroxy-butyl)-1-(3,3,3-trifluor-2-hydroxy-2-methyl-propionyl)-harnstoff;

20. Verbindungen nach einem der Ansprüche 1 bis 19, **dadurch gekennzeichnet, daß** sie ausgewählt sind unter:
- (4-Cyano-3-trifluormethyl-phenyl)-(3,3,3-trifluor-2-hydroxy-2-methyl-propionyl)-carbamidsäure;
- (4-Cyano-3-trifluormethyl-phenyl)-(3,3,3-trifluor-2-hydroxy-2-methyl-propionyl)-carbamidsäureethylester; und
- 1-(3,4-Dichlor-phenyl)-3-propyl-1-(3,3,3-trifluor-2-hydroxy-2-methyl-propionyl)-harnstoff.

21. Verfahren zur Herstellung der Verbindungen der Formel (I) nach einem der Ansprüche 1 bis 20, **dadurch gekennzeichnet, daß** es einen ersten Schritt, in dem ein Isocyanat in einem wasserfreien Lösungsmittel in Gegenwart eines Amins mit einem Cyanohydrin umgesetzt wird, das erhaltene Gemisch dann mit Salzsäure versetzt wird und das gebildete N-Aryl-2,4-dioxo-oxazolidin-Derivat anschließend mit einem organischen Lösungsmittel extrahiert, getrocknet und chromatographisch an einer Kieselgelsäule gereinigt wird; und einen zweiten Schritt umfaßt, in dem zu der im ersten Schritt hergestellten N-Aryl-2,4-dioxo-oxazolidin-Lösung ein Alkohol oder ein Amin im Überschuß zugegeben wird, die Lösung 1 bis 7 Tage auf 25 bis 70 °C erwärmt und anschließend zur Trockene eingedampft und der Rückstand chromatographisch an Kieselgel gereinigt oder umkristallisiert wird.

22. Zusammensetzung, die mindestens eine Verbindung der Formel (I) nach einem der Ansprüche 1 bis 20 enthält.

23. Zusammensetzung nach Anspruch 22, **dadurch gekennzeichnet, daß** sie in der Kosmetik oder Pharmazie verwendet werden soll.

24. Kosmetische Zusammensetzung nach einem der Ansprüche 22 oder 23, **dadurch gekennzeichnet, daß** sie mindestens eine Verbindung der Formel (I) in einer Menge im Bereich von 0,0001 bis 5 % des Gesamtgewichts der Zusammensetzung und vorzugsweise in einer Menge von 0,001 bis 2 % des Gesamtgewichts der Zusammensetzung enthält.

25. Pharmazeutische Zusammensetzung nach einem der Ansprüche 22 oder 23, **dadurch gekennzeichnet, daß** sie mindestens eine Verbindung der Formel (I) in einer Menge im Bereich von 0,001 bis 10 % des Gesamtgewichts der Zusammensetzung und vorzugsweise in einer Menge von 0,01 bis 5 % des Gesamtgewichts der Zusammensetzung enthält.

26. Zusammensetzung nach einem der Ansprüche 22 bis 25, **dadurch gekennzeichnet, daß** sie ferner mindestens ein Mittel enthält, das unter den antibakteriellen Mitteln, antiparasitären Mitteln, Antimykotika, antiviralen Mitteln, entzündungshemmenden Mitteln, Mitteln gegen Juckreiz, Anästhetika, Keratolytika, Mitteln gegen freie Radikale, Antiseborrhöika, Antischuppenmitteln, Mitteln gegen Akne und/oder Mitteln, die die Differenzierung und/oder Proliferation und/oder Pigmentierung der Haut vermindern, und Extrakten pflanzlicher oder bakterieller Herkunft ausgewählt ist.

27. Zusammensetzung nach einem der Ansprüche 22 bis 26, **dadurch gekennzeichnet, daß** sie zur Induzierung und/oder Stimulation des Haarwachstums und/oder zur Verlangsamung des Haarausfalls und/oder zur Behandlung von Hyperseborrhoe und/oder Akne dienen soll.

28. Verbindungen der allgemeinen Formel (I): 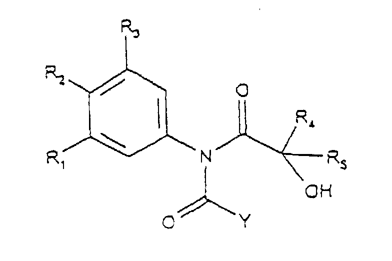 nach den Ansprüchen 1 bis 20 als Arzneimittel.

29. Verwendung einer wirksamen Menge mindestens einer Verbindung der Formel (I) nach einem der Ansprüche 1 bis 20 in einer kosmetischen Zusammensetzung oder zur Herstellung einer pharmazeutischen Zusammensetzung, wobei die Verbindung oder die Zusammensetzungen zur Induzierung und/oder Stimulation des Haarwachstums und/oder zur Verlangsamung des Haarausfalls und/oder zur Behandlung von Hyperseborrhoe und/oder Akne vorgesehen sind.

30. Verfahren zur kosmetischen Behandlung der Haare und/oder der Kopfhaut, **dadurch gekennzeichnet, daß** es darin besteht, daß auf die Haare und/oder die Kopfhaut und/oder die Haut eine kosmetische Zusammensetzung nach einem der Ansprüche 22 bis 24 oder 26 oder 27 aufgetragen wird, diese mit den Haaren und/oder der Kopfhaut und/oder der Haut in Kontakt belassen wird und gegebenenfalls gespült wird.

## Claims

1. Compounds corresponding to the general formula (I): 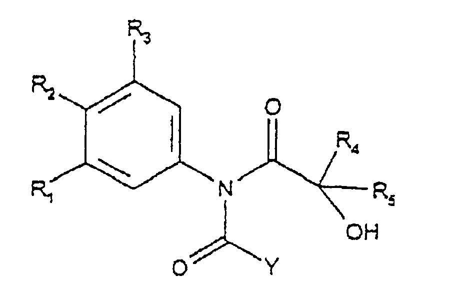 in which
R₁ represents a hydrogen atom, a halogen atom, a cyano group (-CN), a C₁-C₄ alkyl radical, a perfluoroalkyl radical or an -OR₆ radical, R₆ representing a hydrogen atom or a C₁-C₄ alkyl radical;
R₂ represents a nitro group (-NO₂), a cyano group (-CN), a halogen atom, an -SO₂R₇ radical, a -COR₇ radical or a -COOR₇ radical, R₇ representing a hydrogen atom, a C₁-C₄ alkyl radical or a phenyl radical optionally substituted by at least one halogen atom;
R₃ represents a hydrogen atom, a halogen atom, a cyano group (-CN), a C₁-C₄ alkyl radical, optionally substituted by at least one halogen atom, or an -OR₆ radical, R₆ representing a hydrogen atom or a C₁-C₄ alkyl radical;
R₄ represents a hydrogen atom or a C₁-C₄ alkyl radical, optionally substituted by at least one halogen atom;
R₅ represents a C₁-C₄ alkyl radical substituted by at least one halogen atom or a phenyl radical optionally substituted by at least one halogen atom, a cyano group (-CN) or a nitro group (-NO₂);
Y represents an OR radical or an NRR' radical;
R and R', which are identical or different, represent a hydrogen atom, a C₁-C₈ alkyl radical, optionally substituted by at least one hydroxyl radical (-OH), or a -COOR₆ radical; in the case where Y represents NRR', R and R' can form a 5- or 6-membered ring,
or their optical and/or geometrical isomers, alone or as a mixture in all proportions, their acylated forms or their pharmaceutically acceptable salts.

2. Compounds according to the preceding claim, **characterized in that** R₁ is a chlorine atom.

3. Compounds according to Claim 1, **characterized in that** R₁ is preferably a methyl radical.

4. Compounds according to Claim 1, **characterized in that** R₁ is a trifluoromethyl radical (-CF₃).

5. Compounds according to Claim 1, **characterized in that** R₁ is a methoxy radical (-OCH₃).

6. Compounds according to any one of the preceding claims, **characterized in that** R₂ is a chlorine atom.

7. Compounds according to any one of Claims 1 to 5, **characterized in that** R₂ is an -SO₂R₇ radical in which R₇ is a phenyl radical.

8. Compounds according to any one of Claims 1 to 5, **characterized in that** R₂ is a -COR₇ radical in which R₇ is a phenyl radical.

9. Compounds according to any one of Claims 1 to 5, **characterized in that** R₂ is a -COOR₇ radical in which R₇ is an ethyl radical.

10. Compounds according to any one of the preceding claims, **characterized in that** R₃ is a chlorine atom.

11. Compounds according to any one of Claims 1 to 9, **characterized in that** R₃ is a trifluoromethyl radical (-CF₃).

12. Compounds according to any one of Claims 1 to 9, **characterized in that** R₃ is an -OR₆ radical in which R₆ is a methyl radical (-CH₃).

13. Compounds according to any one of the preceding claims, **characterized in that** R₄ is a methyl radical (-CH₃).

14. Compounds according to any one of Claims 1 to 12, **characterized in that** R₄ is a trifluoromethyl radical (-CF₃).

15. Compounds according to any one of the preceding claims, **characterized in that** R₅ is a trifluoromethyl radical (-CF₃).

16. Compounds according to any one of the preceding claims, **characterized in that** Y is an -NHR radical.

17. Compounds according to any one of the preceding claims, **characterized in that** R is a butyl radical.

18. Compounds according to any one of Claims 1 to 16, **characterized in that** R is a -COOR₆ radical in which R₆ is a propyl radical.

19. Compounds according to any one of Claims 1 to 18, chosen from:
- (4-cyano-3-(trifluoromethyl)phenyl)(3,3,3-trifluoro-2-hydroxy-2-methylpropionyl)carbamic acid;
- the ethyl ester of (4-cyano-3-(trifluoromethyl)-phenyl)(3,3,3-trifluoro-2-hydroxy-2-methylpropionyl)-carbamic acid;
- 1-(3,4-dichlorophenyl)-3-propyl-1-(3,3,3-trifluoro-2-hydroxy-2-methylpropionyl)urea;
- 1-(3,4-dichlorophenyl)-3-benzyl-1-(3,3,3-trifluoro-2-hydroxy-2-methylpropionyl)urea;
- 1-(3,4-dichlorophenyl)-3-piperidinyl-1-(3,3,3-trifluoro-2-hydroxy-2-methylpropionyl)urea;
- 1-(4-sulphonylphenyl)-3-propyl-1-(3,3,3-trifluoro-2-hydroxy-2-methylpropionyl)urea;
- 1-(4-benzoylphenyl)-3-propyl-1-(3,3,3-trifluoro-2-hydroxy-2-methylpropionyl)urea;
- the ethyl ester of (3,4-dichlorophenyl) (3,3,3-trifluoro-2-hydroxy-2-methylpropionyl)carbamic acid;
- 1-(3,4-dichlorophenyl)-3-(4-hydroxybutyl)-1-(3,3,3-trifluoro-2-hydroxy-2-methylpropionyl)urea.

20. Compounds according to any one of Claims 1 to 19, chosen from:
- (4-cyano-3-(trifluoromethyl)phenyl)(3,3,3-trifluoro-2-hydroxy-2-methylpropionyl)carbamic acid;
- the ethyl ester of (4-cyano-3-(trifluoromethyl)-phenyl)(3,3,3-trifluoro-2-hydroxy-2-methylpropionyl)-carbamic acid;
- 1-(3,4-dichlorophenyl)-3-propyl-1-(3,3,3-trifluoro-2-hydroxy-2-methylpropionyl)urea.

21. Process for the preparation of the compounds of formula (I) as defined according to any one of Claims 1 to 20, **characterized in that** it comprises a first stage in which an isocyanate is reacted with a cyanohydrin in an anhydrous solvent in the presence of an amine, the mixture obtained subsequently being treated in the presence of hydrochloric acid, the N-aryl-2,4-dioxooxazolidine derivative thus formed then being extracted using an organic solvent and then dried and purified by chromatography on a silica column;
a second stage in which an excess of alcohol or of amine is added to a solution of N-aryl-2,4-dioxooxazolidine obtained in the first stage, the solution then being heated between 25°C and 70°C for 1 to 7 days and then evaporated to dryness, the residue obtained being purified by chromatography on a silica column or recrystallized.

22. Composition comprising at least one compound of formula (I) as defined according to any one of Claims 1 to 20.

23. Composition according to Claim 22, **characterized in that** it is intended for a cosmetic or pharmaceutical use.

24. Cosmetic composition according to either one of Claims 22 and 23, **characterized in that** it comprises at least one compound of formula (I) in an amount representing from 0.0001% to 5% of the total weight of the composition and preferably in an amount representing from 0.001% to 2% of the total weight of the composition.

25. Pharmaceutical composition according to either one of Claims 22 and 23, **characterized in that** it comprises at least one compound of formula (I) in an amount representing from 0.001% to 10% of the total weight of the composition and preferably in an amount representing from 0.01% to 5% of the total weight of the composition.

26. Composition according to any one of Claims 22 to 25, **characterized in that** it also comprises at least one agent chosen from antibacterial agents, agents for combating parasites, antifungals, antivirals, anti-inflammatories, antipruritics, anaesthetics, keratolytics, agents for combating free radicals, antiseborrhoeics, antidandruff agents, antiacne agents and/or agents which decrease cutaneous pigmentation and/or proliferation and/or differentiation, or extracts of plant or bacterial origin.

27. Composition according to any one of Claims 22 to 26, **characterized in that** it is intended to induce and/or stimulate hair growth and/or slow down hair loss and/or to treat hyperseborrhoea and/or acne.

28. As medicaments, the compounds of general formula (I) 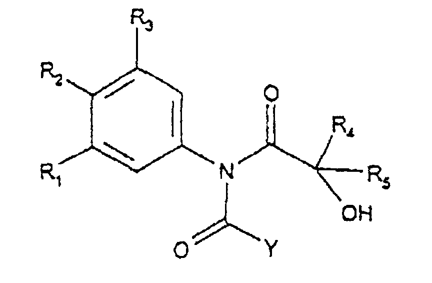 as defined in Claims 1 to 20.

29. Use, in a cosmetic composition or for the preparation of a pharmaceutical composition, of an effective amount of at least one compound corresponding to the general formula (I) as defined according to any one of Claims 1 to 20, this compound or this composition being intended to induce and/or stimulate hair growth and/or slow down hair loss and/or treat hyperseborrhoea and/or acne.

30. Process for the cosmetic treatment of the hair and/or scalp and/or skin, **characterized in that** it consists in applying, to the hair and/or scalp and/or skin, a cosmetic composition as defined in any one of Claims 22 to 24 or 26 or 27, in leaving the composition in contact with the hair and/or scalp and/or skin, and optionally in rinsing.
